**Europäisches Patentamt**

**European Patent Office**

**Office européen des brevets**

(19)

(11) Veröffentlichungsnummer: **0 045 367**

**A1**

(12) ## EUROPÄISCHE PATENTANMELDUNG

(21) Anmeldenummer: **81104624.2**

(22) Anmeldetag: **16.06.81**

(51) Int. Cl.³: **A 61 M 5/00**
**A 61 M 5/31**

(30) Priorität: **01.08.80 DE 3029226**

(43) Veröffentlichungstag der Anmeldung:
**10.02.82 Patentblatt 82/6**

(84) Benannte Vertragsstaaten:
**AT BE CH FR GB IT LI LU NL SE**

(71) Anmelder: **Fürther, Günter**
**Oberfürberger Strasse 26**
**D-8510 Fürth Bayern(DE)**

(72) Erfinder: **Fürther, Günter**
**Oberfürberger Strasse 26**
**D-8510 Fürth Bayern(DE)**

(74) Vertreter: **Göbel, Matthias**
**Pruppacher Hauptstrasse 5-7**
**D-8501 Pyrbaum-Pruppach(DE)**

(54) **Diabetiker-Set.**

(57) Bei einem Diabetiker-Set mit einem Gehäuse zur Aufnahme von wenigstens einer Insulinflasche und einer Spritze sowie gegebenenfalls von Alkoholtüchern ist das Gehäuse durch einen Aufnahmeteil (1) und einen dieses kappenartig übergreifenden, abziehbaren Deckel (2) gebildet, wobei der Aufnahmeteil (1) mit Halterungen für die Insulinflasche derart gestaltet ist, daß die lösbar neben der Insulinflasche (5, 6) gehalterte Spritze nach ihrer Abnahme, ohne daß hierzu ein Ablegen des Gehäuses oder Teilen desselben erforderlich ist, in die in ihrer Halterung verbleibende Insulinflasche (5, 6) einsteckbar ist.

FIG. 4

EP 0 045 367 A1

DIPL.-ING. **M. GÖBEL**
PATENTANWALT

8501 PYRBAUM-R 0045367
PRUPPACHER HAUPTSTRASSE 5-7
TELEFON 09180/675
TELEGRAMM GOEPATENT PYRBAUM
TELEX 624407 GOEPA

BANKKONTEN
VOLKSBANK NÜRNBERG 45233 BLZ 76090000
COMMERZBANK NÜRNBERG 6300907 BLZ 76040061

12. JUNI 1981

- 1 -

Günter Fürther,  D 8510  Fürth/B.

Diabetiker-Set

Die Erfindung bezieht sich auf ein Diabetiker-Set mit einem druckfesten Gehäuse zur Aufnahme wenigstens einer Insulinflasche, einer Spritze sowie gegebenenfalls von Alkoholtüchern od.dgl.

Ein derartiger, vom Anmelder selbst entwickelter Diabetiker-Set hat den Vorteil, daß der Diabetiker jederzeit und ohne allzu großen räumlichen Aufwand alles Notwendige mit sich führen kann, was er braucht, um sich eine oder mehrere Insulinspritzen geben zu können. Auf diese Weise sind Diabetiker auf Reisen unabhängiger und können - was in sehr vielen Fällen der Fall ist - auch ohne weiteres in Restaurants essen, da sie sich anschließend durch das mitgeführte Diabetiker-Set sofort die notwendige Insulininjektion verabreichen können.

Alle bislang bekanntgewordenen derartigen Diabetiker-Sets bestehen im wesentlichen aus einem Kasten, dessen Deckwand als wegklappbarer Scharnierdeckel ausgebildet ist. Im Innern des Kastens sind Klemmhalterungen für die Spritzen und die Insulinflaschen vorgesehen. Um sich nun eine Spritze geben zu können, muß der Benutzer eine Insulinflasche sowie eine Spritze aus der Klemmhalterung im Kasten herausnehmen, der zu diesem Zweck auf einer Ablage abgestellt werden muß. Nachdem das Spritzen von Insulin außerhalb der Wohnung häufig nur in Toiletten, Waschräumen oder an ähnlichen Stellen erfolgen kann, ist diese Art der Handhabung außerordentlich erschwert, da,abgesehen von dem häufigen Fehlen einer Ablage zum Abstellen des Kastens, die hygienischen Verhältnisse in den meisten Fällen nicht dazu angetan sind, daß man den Kasten aus der Hand geben möchte.Dies ist aber zum Spritzen unbedingt erforderlich.

Der Erfindung liegt daher die Aufgabe zugrunde, einen Diabetiker-Set der eingangs genannten Art derart weiterzubilden, daß eine Bedienung möglich ist, die ein Ausderhandlegen des Gehäuses oder von Teilen des Gehäuses völlig entbehrlich machen, so daß unter allen Umständen und unter Wahrung der bestmöglichen hygienischen Voraussetzungen der Benutzer eines derartigen Diabetiker-Sets sich eine Insulininjektion geben kann. Darüber hinaus soll der Diabetiker-Set so ausgestaltet sein, daß er nicht nur selbst möglichst bruchfest ist, sondern daß auch bei einem Herunterfallen keine Gefahr einer Beschädigung der Spritzen oder Insulinflaschen besteht.

Zur Lösung dieser Aufgabe ist erfindungsgemäß vorgesehen, daß das Gehäuse mit einem kappenartig das Aufnahmeteil übergreifenden abziehbaren Deckel versehen ist und daß das Aufnahmeteil mit seinen Halterungen so ausgebildet ist, daß die lösbar neben der Insulinflasche gehalterte Spritze nach ihrer Aufnahme in die in ihrer Halterung

verbleibende Insulinflasche einsteckbar ist.

Der erfindungsgemäß kappenartig ausgebildete Deckel an Stelle eines flachen Klappdeckels ermöglicht zum einen die Ausbildung des Aufnahmeteils derart, daß es nach wenigstens einer Seite hin offen ist, so daß zum Einführen der Spritze in die Insulinflasche diese nicht extra aus dem Aufnahmeteil herausgenommen zu werden braucht. Darüber hinaus ergibt sich durch diese kappenartige Ausbildung eine sehr viel größere Steifigkeit und damit Bruchfestigkeit der gesamten Anordnung.

Bevorzugt sollen die Insulinflaschen mit allseitig freiliegenden Köpfen im Innern des Gehäuses gehaltert sein. so daß selbst ein etwaiges Ablegen des Deckels bei der Anwendung vom hygienischen Standpunkt keinerlei Beeinträchtigung mit sich bringen kann. Darüber hinaus kann aber selbstverständlich vorgesehen sein, daß der Deckel entweder durch ein Verbindungsband mit dem Unterteil verbunden bleibt, so daß er einfach herunterhängt, nachdem man ihn abgezogen hat, oder aber,daß man ihn von der entgegengesetzten Seite auf das Fußende des Aufnahmeteils aufsteckt, während man an dem freiliegenden oberen Ende die Spritze an die Insulinflasche ansetzt.

Die Halterungen für die Insulinflaschen sollen in Ausgestaltung der Erfindung so ausgestaltet sein, daß Flaschen unterschiedlichen Durchmessers und/oder Länge gehaltert werden können. Dabei braucht der Variationsbereich gar nicht allzu groß gehalten zu werden, da die handelsüblichen Insulinflaschen - dies gilt in jedem Fall für die marktführenden Fabrikate - sich nur um einige Millimeter im Durchmesser unterscheiden. Beispielsweise durch Ausbildung der Halterung als seitlich geschlitzte Hülsenabschnitte aus einem elastisch federnden Material, insbesondere Kunststoff, lassen sich somit mit einem Typ oder allenfalls mit wenigen Grundtypen alle gängigen Insulinflaschen in einem erfindungsgemäßen Set unterbringen.

Gemäß einer besonders vorteilhaften Ausführungsform der vorliegenden Erfindung kann das Gehäuse nach Art eines querschnittlich ovalen Stecketuis für Brillen ausgebildet sein, wobei das Aufnahmeteil zumindest am oberen Ende querschnittlich im wesentlichen hantelförmig ausgebildet ist. Dieser Querschnitt ergibt sich dabei durch zwei seitliche Einsteckhülsen für die Insulinflaschen und einer sie verbindenden Stegwand mit Schnapphalterungen für die Spritzen.

Die gängigen Insulinflaschen sind relativ kurz und gedrungen, in jedem Fall wesentlich kürzer als die Spritzen. Gemäß eine Weiterbildung der Erfindung sollen die Einsteckhülsen deshalb verlängert ausgebildet sein, derart, daß unter jeder Insulinflasche eine Reserveflasche Platz findet. Das Vorsehen von zwei seitlichen Einsteckhülsen für Insulinflaschen, die beide nach dem Abziehen des Deckels wahlweise zugängig sind, hat nicht nur den Zweck, um einen größeren Vorrat mitführen zu können, sondern ermöglicht die vorteilhafte Verwendung des erfindungsgemäßen Sets auch für solche Personen, die zwei unterschiedliche Insulinsorten gleichzeitig spritzen müssen.

Bei dem genannten brillenetuiartig ausgebildeten Gehäuse ist es besonders vorteilhaft, wenn die Stegwand etwa in der Mittelebene der Einsteckhülsen liegt und der kappenartige Deckel relativ lang ausgebildet ist, so daß am unteren Ende des Aufnahmeteils nur eine kurze ovale Hülse vorgesehen zu sein braucht. Dies bedeutet, daß die Stegwand den größten Teil ihrer Länge nach dem Abziehen des Deckels frei heraussteht, so daß die Spritzen nicht aus engen Kanälen herausgezogen zu werden brauchen, sondern nur mit ihrem Ende in die kurze Hülse einragen und zu ihrem Abnehmen und Wiederaufsetzen ein einfaches Wegschwenken nach vorne bzw.nach hinten erforderlich ist. Zur Verhinderung eines Abziehens der Schutzkappen von den Spritzen sollen dabei die Aufnahmen für die beiden Spritzen durch an der Stegwand befestigte Abstandshalter, beispielsweise eine Längsrippe, voneinander getrennt sein. Die Spritzen liegen geschützt in einer durch die seitlichen Einsteckhülsen für die Insulinflaschen gebildeten Mulde, so daß die Gefahr eines Zerbrechens selbst dann nicht besteht, wenn das Gehäuse auf den Boden fallen sollte, ja nicht einmal dann, wenn zu diesem Zeitpunkt bereits der kappenartige Deckel abgezogen ist. Durch die parallel zur mittigen Stegwand liegenden Wände des unteren ovalen Hülsenabschnitts des Aufnahmeteils ergibt sich nicht nur der Aufnahmeraum für die Enden der Spritzen, sondern auch auf der gegenüberliegenden Seite ein Aufnahmeraum für Alkoholtücher zum Desinfizieren der Einstichstellen am Körper des Benutzers.

Mit besonderem Vorteil sollen die Halterungen für die Insulinflaschen so ausgebildet sein, daß in ihrer Betriebsstellung die Insulinflaschen teilweise aus den Halterungen herausragen, so daß die Etiketten teilweise sichtbar sind. Dies ist besonders wichtig für Diabetiker, die jeweils zwei unterschiedliche Insuline gleichzeitig benutzen müssen, damit diese auf einen Blick erkennen, welches Insulin in welcher Flasche angeordnet ist, da üblicherweise die notwendigen Anteile der beiden Sorten nicht gleichgroß sind. Um dennoch einen besonders hohen Schutz gegen eine Beschädigung, auch bei unvorsichtiger Handhabung zu gewährleisten, sollen die Insulinflaschen in ihren Halterungen dann einseitig durch einen Wandabschnitt nach oben überragt werden.

Anstelle des genannten brilletuiartigen Gehäuses mit einer relativ langen Kappe kann das Gehäuse auch beispielsweise seifenschalenartig mit den Zugang zu den Insulinflaschen freigebenden Ausnehmungen in den Seitenwänden ausgebildet sein. Beispielsweise können diese Seitenwände praktisch völlig weggelassen sein, während die Längsseitenwände zumindest im mittleren Bereich des Aufnahmeteils in jedem Fall verbleiben sollten, um eine bessere Handhabung bei der Benutzung sicherzustellen. Diese seifenschalenartige Grundform des Gehäuses mit der Möglichkeit, Ausnehmungen in den beiden gegenüberliegenden Seitenwänden vorsehen zu können, ermöglicht es in der Längsmittellinie des Aufnahmeteils Halterungen für zwei gegeneinandergestellte Insulinflaschen vorzusehen, während Klemmhalterungen für die Spritzen links und rechts seitlich neben der Halterungseinrichtung für die Flaschen angeordnet sind. Die Unterbringung der Alkoholtücher erfolgt in diesem Fall auf der Innenseite des Deckels, an dem entsprechende Befestigungseinrichtungen, beispielsweise in Form eines einfachen die Tücher übergreifenden und an einer Seite aushakbaren Spanngummis vorgesehen sind. Auch in diesem Fall kann die Ausbildung wieder so getroffen sein, daß sich der Deckel nach dem Abnehmen auf die Unterseite des Aufnahmeteils aufstecken läßt, so daß er zur B-enutzung des Sets ebenfalls nicht aus der Hand gegeben zu werden braucht.

Diese besondere Ausbildung des Aufnahmeteils mit fehlenden Stirnseiten , so daß von gegenüberliegenden Seiten aus Insulinflaschen ohne Abnahme von Halterungsteil zugängig sind, ermöglicht auch eine weitere im Gebrauch besonders vorteilhafte Ausbildungsmöglichkeit mit unverlierbarem Deckel. Zu diesem Zweck ist in Ausgestaltung der Erfindung vorgesehen, daß der Deckel als an einer Längsschmalseite offener Flachquader ausgebildet ist, aus dem das im wesentlichen eine Platte mit der angeformten fehlenden Quaderwand ausgebildete Aufnahmeteil seitlich herausziehbar ist. Dabei können Führungen für die Gleitlagerungen des Aufnahmeteils vorgesehen sein, beispielsweise in Form von die offenen Stirnkanten der Platte aufnehmenden Nuten in den parallel zur Ausgangsrichtung liegenden Stirnwänden des Deckels, oder auch in Nuten in den zur Platte parallelen Deckwänden,in welchen rudimentäre Stirnwandstege des Aufnahmeteils geführt sind.

Dabei sollen mit besonderem Vorteil Auszugsarretierungen vorgesehen sein, die eine vollständige Trennung des Deckels vom Aufnahmeteil verhindern.

Die erfindungsgemäße Ausbildung eines Diabetiker-Sets, welches im Prinzip aus beliebigen Werkstoffen hergestellt werden kann, ergibt die besonders vorteilhafte Möglichkeit der Fertigung des Unterteils und des Deckels des Gehäuses als jeweils einstückige Formteile aus Kunststoff, wobei es gleichwohl möglich ist, durch eine besonders lederartige Narbung der Oberfläche ein gefälliges wertvolles Aussehen dieser Kunststoffteile sicherzustellen. Dabei liegt es auch im Rahmen der Erfindung, geschäumte Kunststoffe, beispielsweise Polyurethanschaum, zu verwenden, die bei sehr geringem Gewicht eine hohe Bruchfestigkeit ergeben.

Weitere Vorteile, Merkmale und Einzelheiten der Erfindung ergeben sich aus der nachfolgenden Beschreibung eines Ausführungsbeispiels sowie an Hand der Zeichnung. Dabei zeigen:

Fig. 1    eine teilweise aufgebrochene Ansicht einer ersten Ausführungsform eines erfindungsgemäßen Diabetiker-Sets mit  abgenommener Deckel-Kappe,

Fig.2    eine teilweise geschnittene Seitenansicht der Anordnung nach Fig. 1,

Fig.3    eine Draufsicht auf das Aufnahmeteil des Sets, in Richtung des Pfeils P in Fig. 1,

Fig. 4 und 5 eine Vorderansicht und eine teilweise aufgebrochene Seitenansicht auf eine zweite Ausführungsform eines erfindungsgemäßen Diabetiker-Sets,

Fig. 6    einen Schnitt längs der Linie VI-VI in Fig. 4,

Fig. 7 und 8 eine Ansicht des Aufnahmeteils und eine Seitenansicht mit abgenommenem Deckel,

Fig. 9    einen Schnitt längs der Linie IX-IX  in Fig. 7,

Fig.10    einen Schnitt parallel zur Auszugsrichtung durch eine vierte Ausführungsform eines erfindungsgemäßen Diabetiker-Sets mit aus der Kappe herausziehbaren Aufnahmeteil und

Fig.11    einen Längsschnitt längs der Linie XI-XI in Fig.lo.

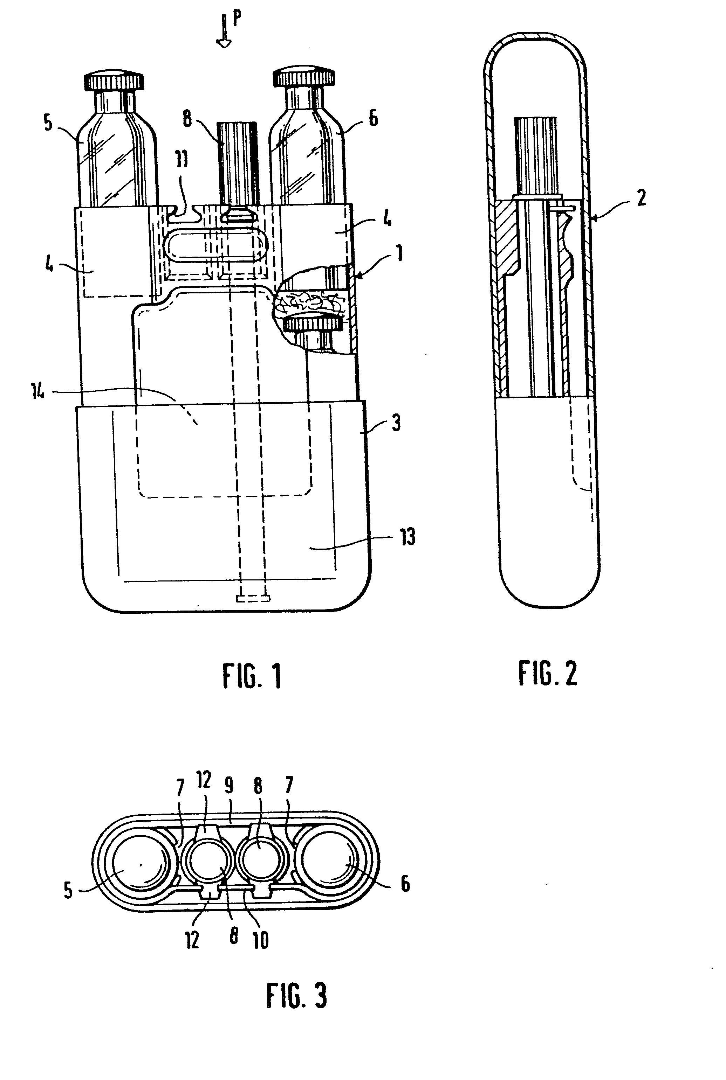

Das nach Art eines Brillenetuis ausgebildete Diabetiker-Set gemäß den Fig. 1-3 besteht aus dem Unterteil 1 und einem aufsteckbaren kappenartigen Deckel 2, der das Unterteil sehr weit übergreift, so daß das in der Form dem kappenartigen Deckel 2 entsprechende Unterteil 3 des Aufnahmeteils 1 relativ niedrig ist. Das Aufnahmeteil umfaßt zwei seitliche Einsteckhülsen 4 zur Aufnahme von Insulinflaschen 5 und 6, wobei zur klemmenden Halterung dieser Insulinflaschen 5 und 6 sowie zur Anpassung an unterschiedliche Flaschendurchmesser die Einsteckhülsen bei 7 geschlitzt sind, so daß sie federnd aufweitbar sind. Zur Halterung der relativ kurzen Insulinflaschen 5 und 6 bräuchten die Einsteckhülsen nicht so lange ausgebildet werden wie das Unterteil selbst, dessen Länge im wesentlichen durch die Länge der Spritzen 8 bestimmt wird, die zwischen den Einsteckhülsen 4 für die Insulinflaschen 5 und 6 lösbar im Unterteil 1 gehaltert sind. Zu diesem Zweck sind beim Ausführungsbeispiel nach den Fig. 1 - 3 die Einsteckhülsen 4 durch eine rückwärtige Wand 9 und eine gegenüber der tangentialen Vorderebene der Einsteckhülsen 4 zurückgesetzte Vorderwand 10 verbunden, so daß zwischen ihnen ein, gegebenenfalls durch eine Querwand nochmals aufgeteilter Aufnahmeraum für die Spritzen entsteht. Hinterschnittene Nutausnehmungen 11 in den Wänden 9 und 10 ermöglichen ein Einrasten der der Handhabung der Spritzen dienenden Queransätze 12, die normalerweise bei der Betätigung der Spritze auf Zeigefinger und Mittelfinger aufliegen. Ebenso wie es ausreicht, wenn zwischen den Wänden 9 und 10 nur kurze Führungsaufnahmen für die Spritzen vorgesehen sind, wie es in Fig. 1 gestrichelt angedeutet ist, da dann in jedem Fall sichergestellt ist, daß sich die beiden Spritzen weder beim Einstecken noch beim Herausziehen behindern können, bräuchten auch die Einsteckhülsen 4 für die Insulinspritzen 5 und 6 nicht über die gesamte Länge durchzugehen. Bildet man sie aber durchgehend aus, so hat dies den Vorteil, daß hinter jeder vorne aus dem Aufnahmeteil herausstehenden Flasche 5 und 6 eine weitere Vorratsflasche angeordnet sein kann. Durch das Zurückspringen der Vorderwand 10 gegenüber der Vorderebene 13 des Unterteils 3 des Halterungsteils 1, wobei diese Ebene mit der Tangentialebene an die beiden Einsteckhülsen 4 zusammenfällt, ergibt sich ein Aufnahmeraum 14 zum Einstecken von Alkoholtüchern. Diese Alkoholtücher werden benötigt, um die Einstichstelle am Körper des Benutzers jeweils keimfrei zu machen.

Die erfindungsgemäße Ausbildung des Diabetiker-Sets macht es nicht notwendig, daß das Unterteil zur Handhabung irgendwo abgelegt wird oder daß die Insulinflasche überhaupt aus ihrer Halterung entfernt wird. Man entnimmt eine Spritze 8 aus ihrer federnd verrasteten Einsteckstellung und kann sie anschließend in Richtung des Pfeils P in die eine und/oder andere Insulinflasche einstechen und aufziehen. Nach dem Spritzen wird die Spritze wieder in ihre Aufnahme eingesteckt und die Deckel-Kappe 2 aufgeschoben. Neben der Kleinvolumigkeit und damit leichten Mitführbarkeit und Handhabbarkeit des erfindungsgemäßen Diabetiker-Sets ergibt die gezeigte Et-uiausbildung auch eine sehr große Steifigkeit und Druckfestigkeit, so daß die Gefahr praktisch völlig ausgeschaltet ist, daß eine Insulinflasche oder eine Spritze zerbrechen kann, selbst wenn das Set einmal auf den Boden fallen sollte. Dies ist besonders wichtig, da ein Diabetiker, dem sein Diabetiker-Set unterwegs kaputtgeht, beispielsweise im Urlaub - seine Reise abbrechen und wieder nach Hause fahren muß.

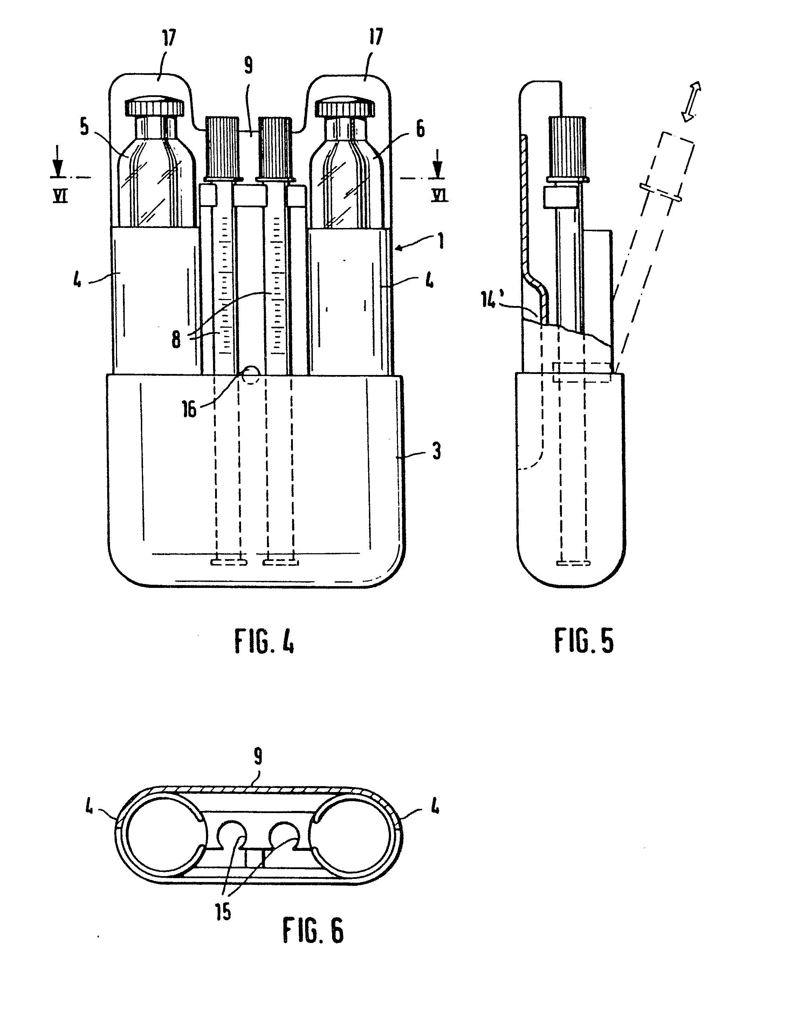

Bei der prinzipiell in gleicher Weise brilletuiartig ausgebildeten Ausführungsform nach den Fig. 4 - 6 ist die Vorderwand 10 im Verbindungsab-schnitt zwischen den beiden Einsteckhülsen 4 weggelassen, so daß die Spritzen unmittelbar vor der Hinterwand 9 in entsprechenden, ein Lösen durch Verschwenken nach vorne ermöglichenden Rastausnehmungen 15 gehaltert sind. Die Trennung der Aufnahmen für die beiden Spritzen 8 ist bei dieser Ausführungsform auf einen einfachen, von der Stegwand 9 zwischen den Einsteckhülsen 4 nach vorne ragenden Bolzen 16 reduziert. Das Einsetzen und Abnehmen erfolgt, wie in Fig. 5 strichpunktiert, angedeutet ist, durch Verschwenken der Spritzen nach vorne bzw. verschwenktes Einsetzen in den durch das Unterteil 3 gebildeten Aufnahmeraum.

Anstelle der Anordnung der Stegwand 10 in der Mittelebene der Einsteckhülsen 4, um auf diese Weise auf beiden Seiten einen Aufnahmeraum für die Enden der Spritzen 8 einerseits sowie einen weiteren Aufnahmeraum 14' für die Alkoholtücher zu schaffen, ist die Wand 9, wie in Fig. 5 besonders gut zu erkennen ist, mit einer Einwölbung versehen, um diesen Aufnahmeraum 14' für die Alkoholtücher zu bilden. Darüber hinaus ist die Stegwand 9 im Bereich der Insulinflaschen 5 und 6 mit sie überragenden Fortsätzen 17 versehen, die einen Schutz der Insulinflaschen gegen Bruch bei der Handhabung bewirken. Die Ausbildung der Einsteckhülsen 4 derart, daß die Insulinflaschen 5 und 6 ein ganzen Stück vorne herausschauen können, ist wesentlich damit Diabetiker, die zwei unterschiedliche Insulinsorten gleichzeitig anwenden müssen, durch das herauscheinende Etikett sofort erkennen können, welches Insulin in welcher Flasche ist.

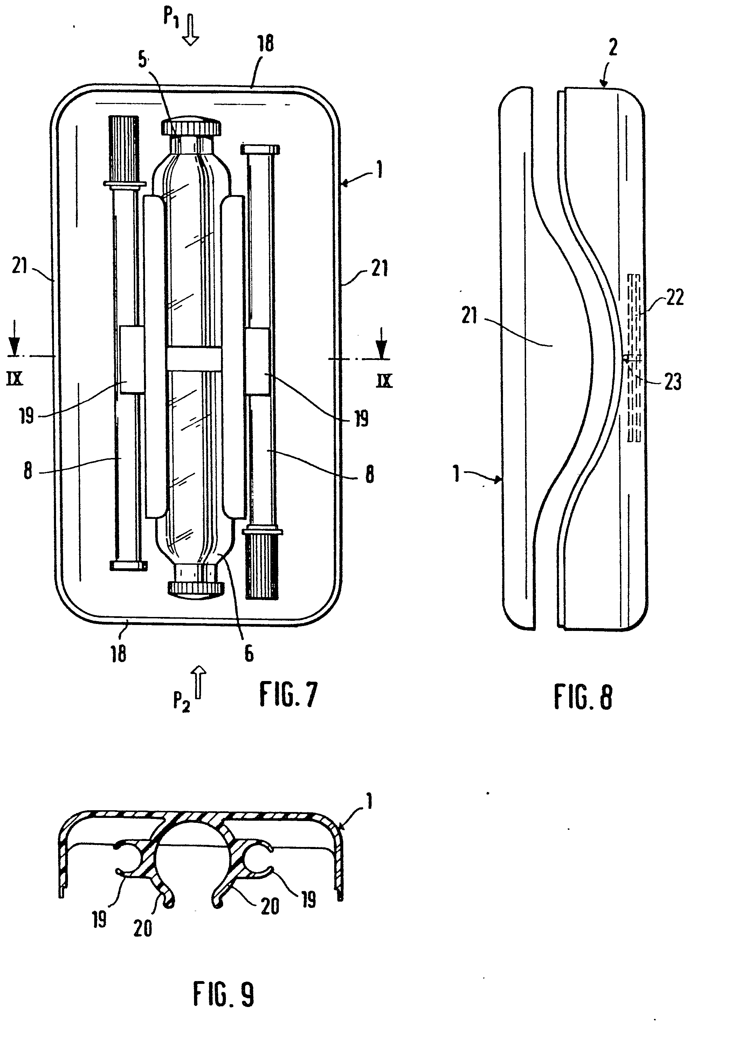

Die erfindungsgemäße Handhabbarkeit des Diabetiker-Sets ohne Herausnahme der Insulinflaschen und ohne Abstellen von wesentlichen Teilen, selbst das Ablegen der Kappe kann dadurch vermieden werden, daß sie durch eine Kordel od.dgl. stets mit dem Unterteil verbunden bleibt, so daß sie während der Handhabung des Sets einfach vom Unterteil herunterhängt, läßt sich, wie in Fig. 7 - 9 dargestellt ist, auch dadurch erreichen, daß man die Stirnwände 18 des Unterteils 1 praktisch völlig wegläßt. Auf diese Weise läßt sich wiederum die Spritze entweder in Richtung des Pfeils $P_1$ von oben oder in Richtung des Pfeils $P_2$ von unten an eine der Insulinflaschen 5 oder 6 ansetzen. Die Halterung der Spritzen liegt in diesem Fall nicht zwischen den Aufnahmen für die Insulinflaschen 5 und 6, wie bei den Ausführungsformen nach den Fig. 1 - 6, sondern seitlich neben ihnen. Beispielsweise können die gabelförmigen Klemmhalterungen 19 für die Spritzen 8 direkt an den Seitenschenkeln 20 der Klemmhalterung für die Insulinflaschen 5 und 6 angeformt sein. Die Längsseitenwände 21 des Aufnahmeteils 1 des Gehäuses sind, wie man besonders gut aus Fig. 8 erkennen kann, zumindest im Mittelteil noch vorhanden, so daß sie besser von der Hand erfaßt werden können. Die komplementär zu den geschwungenen Seitenwänden des Unterteils ausgebildeten Seitenwände des kappenartigen Deckels 2 greifen mit ihren zurückspringenden freien Randkanten nach Art der üblichen Verbindung von Schale und Deckel bei Seifenschal-en in das Aufnahmeteil 1 ein. Zur Befestigung der Alkoholtücher 22 ist eine Halterungsvorrichtung im Innern des Deckels 2 angeordnet, die unter anderem einen Spanngummi 23 umfassen kann, der die Alkoholtücher an der Innenseite klemmend festhält.

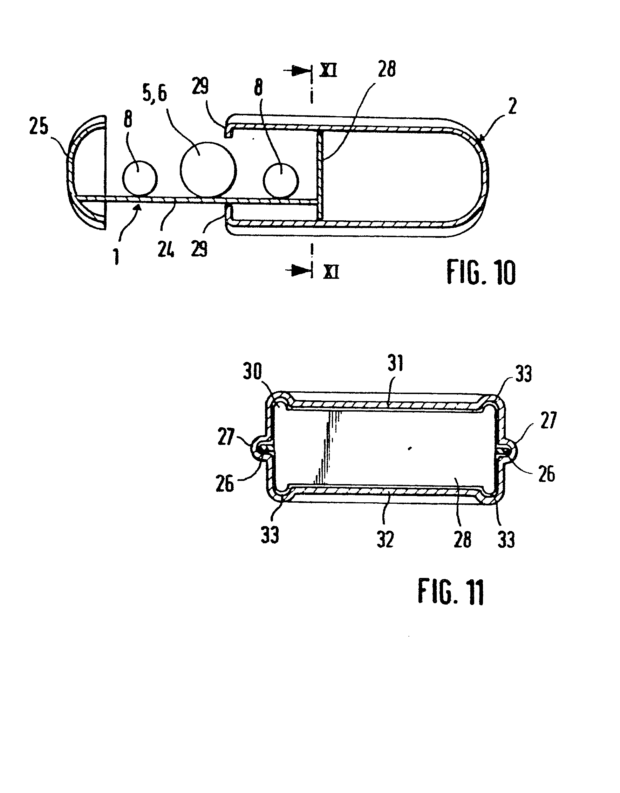

Die schematisch in den Fig. 10 und 11 dargestellte Variante eines erfindungsgemäßen Diabetiker-Sets umfaßt ein Halterungsteil 1, welches aus dem kappenförmigen Deckel 2 herausziehbar ist, so daß dann die nur schematisch angedeuteten, etwa wie in Fig. 7 gezeigt, an der Platte 24 des Aufnahmeteils 1 gehaltenten Insulinflaschen 5, 6 mittels der abgenommenen Spritze 8 von der einen oder anderen parallel zur Zeichenebene liegenden Stirnseiten her angestochen werden können. In der einfachsten Ausführungsform umfaßt das Unterteil 1 lediglich die Platte 24 und die die offene Vorderseite der Kappe 2 verschließende Längsseitenwand 25 sowie selbstverständlich die Halterung für Spritze und die Insulinflaschen sowie gegebenenfalls die Alkoholtücher auf der Unterseite der Seite 24. Die Führung kann dadurch erfolgen, daß die Platte 24 mit seitlichen Randkanten 26 in durch Ausdrückungen 27 gebildete Nuten der Stirnwände der Kappe 2 eingreift. Stattdessen könnte auch eine zusätzliche innere Längsseitenwand 28 vorgesehen sein, die in Verbindung mit einspringenden Randstegen 29 eine Auszugsbegrenzung des Aufnahmeteils darstellt und die mit noppenartigen Verlängerungen 30 in aus den Deckflächen 31 und 32 der Kappe ausgedrückten Rinnen 33 geführt ist. Diese in den Fig. 11 und 12 dargestellte Variante des erfindungsgemäßen Sets ist im Hinblick auf die Zusammenhängigkeit von Aufnahmeteil und Deckel auch in der ausgezogenen Betriebsstellung hinsichtlich der hygienischen Handhabung besonders vorteilhaft und läßt sich dabei auch recht einfach herstellen. Die einfache Herstellbarkeit gilt jedoch im wesentlichen auch für die übrigen Varianten, die sich sämtlich im Wege des Spritzgießens oder Formens aus Kunststoff fertigen lassen, wobei das Aufnahmeteil ebenso wie der Deckel jeweils ein einstückiges Bauteil bilden können. Dies vereinfacht und verbilligt die Fertigung eines erfindungsgemäßen Sets ganz erheblich, wobei durch zusätzliche Oberflächengestaltungen (Narbung) erreicht werden kann, daß ein derartiges Set trotz seiner Spritzgießfertigung aus Kunststoff ein sehr gediegenes wertvolles Aussehen erhält.

Die Erfindung ist nicht auf die dargestellten Ausführungsbeispiele beschränkt. Ersichtlich ließe sich die erfindungsgemäße Ausbildung, bei der der Benutzer in eine Hand eine Spritze nimmt, während er den gesamten Rest des Sets in der anderen Hand behalten kann, ohne irgendwelche Teile abnehmen oder sonstwo abstellen zu müssen, auch noch mit einer Reihe von anderen Ausbildungsformen lösen.

Bei der Ausbildung des Diabetiker-Sets soll im übrigen darauf geachtet werden, daß das Gehäuse möglichst wärmedämmend ist, was beispielsweise durch Verwendung von aufgeschäumtem Kunststoff erreicht werden kann. Darüber hinaus soll es feuchtigkeitsfest und - dicht sein, so daß es zu Hause oder im Hotel im Kühlschrank aufbewahrt werden kann, da Insulin bekanntlich kühl ge-halten werden sollte.

- 1 -

Patentansprüche

1. Diabetiker-Set mit einem druckfesten Gehäuse zur Aufnahme wenigstens einer Insulinflasche, einer Spritze sowie gegebenenfalls von Alkoholtüchern od.dgl., dadurch gekennzeichnet, daß das Gehäuse mit einem kappenartig das Aufnahmeteil (1) übergreifenden, abziehbaren Deckel (2) versehen ist und daß das Aufnahmeteil (1) mit seinen Halterungen so ausgebildet ist, daß die lösbar neben der Insulinflasche (5, 6) gehalterte Spritze (8) nach ihrer Abnahme in die in ihrer Halterung verbleibende Insulinflasche (5, 6) einsteckbar ist.

2. Diabetiker-Set nach Anspruch 1, dadurch gekennzeichnet, daß die Insulinflasche (n) (5, 6) mit allseitig freiliegenden Köpfen im Innern des Gehäuses (1, 2) gehaltert sind.

3. Diabetiker-Set nach Anspruch 1 oder 2, dadurch gekennzeichnet, daß die Halterungen für die Insulinflaschen (5, 6) so ausgebildet sind, daß Flaschen unterschiedlichen Durchmessers und/oder Länge gehaltert werden können.

4. Diabetiker-Set nach Anspruch 3, dadurch gekennzeichnet, daß die Halterungen seitlich geschlitzte Hülsenabschnitte (4) aus einem elastisch federnden Material, insbesondere Kunststoff umfassen.

5. Diabetiker-Set nach einem der Ansprüche 1 bis 4, dadurch gekennzeichnet, daß das Aufnahmeteil mit seitlichen Einsteckhülsen (4) für die Insulinflaschen (5,6) und zwischen ihnen liegenden Halterungen (11, 15) für Spritzen (8) versehen ist.

6. Diabetiker-Set nach Anspruch 5, dadurch gekennzeichnet, daß die Einsteckhülsen (4) so bemessen sind, daß unter jeder Insulinflasche (5, 6) eine Reserveflasche Platz findet.

7. Diabetiker-Set nach einem der Ansprüche 5 oder 6, dadurch gekennzeichnet, daß an einer die Einsteckhülsen (4) verbindenden Stegwand (9) Schnapphalterungen (15) für die Spritzen (8) befestigt, vorzugsweise angeformt sind.

8. Diabetiker-Seit nach Anspruch 7, dadurch gekennzeichnet, daß parallel zur etwa in der Mittelebene der Einsteckhülsen (4) liegenden Stegwand am unteren Ende des Aufnahmeteils (1) Aufnahmeräume (14, 14') für die Spritzenenden bzw. Alkoholtücher bildende Querwände vorgesehen sind.

9. Diabetiker-Set nach einem der Ansprüche 6 bis 8, gekennzeichnet durch die Aufnahmen für zwei Spritzen (9) trennende, an der Stegwand (9) befestige Abstandshalter (16), insbesondere in Form einer Rippe.

10. Diabetiker-Set nach einem der Ansprüche 1 bis 9, dadurch gekennzeichnet, daß die Insulinflaschen (5, 6) in ihren Halterungen einseitig durch einen Wandabschnitt (17) nach oben überragt werden.

11. Diabetiker-Set nach einem der Ansprüche 5 bis 10, dadurch gekennzeichnet, daß das Gehäuse nach der Art eines querschnittlich ovalen Stecketuis für Brillen ausgebildet ist, dessen Aufnahmeteil (1) zumindest am oberen Ende querschnittlich hantelförmig ausgebildet ist.

12. Diabetiker-Set nach einem der Ansprüche 1 bis 4, dadurch gekennzeichnet, daß das Gehäuse seifenschalenartig mit den Zugang zu den Insulinflaschen (5, 6) freigebenden Ausnehmungen in den Seitenwänden ausgebildet ist(Fig.7-9).

BAD ORIGINAL

13. Diabetiker-Set nach Anspruch 12, dadurch gekennzeichnet, daß in der Längsmittelebene des Aufnahmeteils (1) Halterungen für zwei gegeneinandergestellte Insulinflaschen (5, 6) und daneben Klemmhalterungen (19) für Spritzen (8) vorgesehen sind.

14. Diabetiker-Set nach Anspruch 12 oder 13, dadurch gekennzeichnet, daß der Deckel (2) auf seiner Innenseite mit einer Befestigungseinrichtung (23) für Alkoholtücher (22) od.dgl. versehen ist.

15. Diabetiker-Set nach einem der Ansprüche 1 bis 10, dadurch gekennzeichnet, daß der Deckel (2) als an einer Längsschmalseite offener Flachquader ausgebildet ist, aus dem das im wesentlichen eine Platte (24) mit der angeformten fehlenden Quaderwand (25) ausgebildeten Aufnahmeteil (1) seitlich herausziehbar ist (Fig.10 und 11).

16. Diabetiker-Set nach Anspruch 15, gekennzeichnet durch eine vollständige Trennung des Deckels (2) vom Aufnahmeteil (1) verhindernde Auszugsarretierung.

17. Diabetiker-Set nach Anspruch 15 oder 16, dadurch gekennzeichnet, daß das Aufnahmeteil (1) im Querschnitt parallel zur Auszugsrichtung im wesentlichen I-förmig ausgebildet ist.

18. Daibetiker-Set nach einem der Ansprüche μ5 bis 17, gekennzeichnet durch in Führungsrinnen (27, 33) des Deckels (2) eingreifende Führungsstege (26, 30) des Aufnahmeteils (1).

19. Diabetiker-Set nach einem der Ansprüche 1 bis 18, dadurch gekennzeichnet, daß das Aufnahmeteil (1) und der Deckel (2) des Gehäuses jeweils einstücke Formteile aus Kunststoff sind.

P

5    8    6

11    4

4    1

14    3

13

**FIG. 1**

2

**FIG. 2**

7  12  9  8  7

5    6

12  8  10

**FIG. 3**

FIG. 4

FIG. 5

FIG. 6

FIG. 7

FIG. 8

FIG. 9

FIG. 10

FIG. 11

0045367

Nummer der Anmeldung

EP 81 10 4624

Europäisches
Patentamt

**EUROPÄISCHER RECHERCHENBERICHT**

| | EINSCHLÄGIGE DOKUMENTE | | KLASSIFIKATION DER ANMELDUNG (Int Cl ⁹) |
|---|---|---|---|
| Kategorie | Kennzeichnung des Dokuments mit Angabe, soweit erforderlich, der maßgeblichen Teile | betrifft Anspruch | |
| X | <u>US - A - 2 740 516</u> (RENN)<br>* Abbildungen 1-3; Spalte 1, Zeile 43 - Spalte 4, Zeile 8 *<br><br>-- | 1 | A 61 M 5/00<br>5/31 |
| | <u>US - A - 2 174 329</u> (O'NEILL)<br>* Abbildungen 1-3,8; Spalte 1, Zeilen 6-10; Spalte 2, Zeile 49 - Spalte 3, Zeile 12 *<br><br>-- | 1,3,4 | |
| | <u>US - A - 1 625 035</u> (LILLY)<br>* Abbildung; Seite 1, Zeilen 65-68, 98-102; Seite 1, Zeile 109 - Seite 2, Zeile 3 *<br><br>---- | 4,7 | RECHERCHIERTE SACHGEBIETE (Int. Cl.³)<br><br>A 61 B<br>A 61 M |

KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angefuhrtes Dokument

L: aus andern Gründen angefuhrtes Dokument

&: Mitglied der gleichen Patentfamilie, übereinstimmendes Dokument

Der vorliegende Recherchenbericht wurde fur alle Patentansprüche erstellt

| Recherchenort | Abschlußdatum der Recherche | Prufer |
|---|---|---|
| Den Haag | 03-11-1981 | DURAND-SMET |

EPA form 1503.1   06.78